# EUROPEAN PATENT APPLICATION

(11) **EP 4 585 153 A1**
(43) Date of publication of application: **16.07.2025**
(21) Application number: 24151387.8
(22) Date of filing: 11.01.2024
(51) Int. Cl.: A61B 6/00, G01R 33/48

(54) **DETERMINING CONTROL PARAMETERS FOR LIGHT SOURCES**

(71) Applicant: Koninklijke Philips N.V., 5656 AG Eindhoven (NL)
(72) Inventor: KRUEGER, Sascha, Eindhoven (NL); SOMMER, Karsten, Eindhoven (NL)
(74) Representative: Philips Intellectual Property & Standards

(57) **Abstract**

Disclosed herein is a method for determining one or more control parameters (416) for controlling one or more light sources (142) to adjust lighting conditions of a medical imaging system (100) during an acquisition of optical sensor data (414) using an optical sensor (144). The method comprises starting a feedback loop, in response to receiving an adjustment trigger. The feedback loop comprises controlling an adjusting of the lighting conditions using the one or more light sources (142), receiving adjusted optical sensor data (414) for the adjusted lighting conditions from the optical sensor (144), detecting the target elements (122) within the adjusted optical sensor data (414) using a target detection module (412), and determining confidence values for the detections of the target elements (122). The feedback loop is repeated until the confidence values determined for the detections of the target elements (122) reach a predefined confidence threshold level.

## Description

### FIELD OF THE INVENTION

The invention relates to medical imaging, in particular to a method for determining one or more control parameters for controlling one or more light sources to adjust lighting conditions of a medical imaging system during an acquisition of optical sensor data using an optical sensor. The optical sensor is configured for a supervision of the medical imaging system. Furthermore, the invention relates to a computer program comprising machine executable instructions for execution of the method, a computing device for execution of the method, and a medical imaging system comprising the computing device.

### BACKGROUND OF THE INVENTION

In modern scanning environments of medical imaging systems, lighting may be fully accommodated, e.g., to individual preferences of the patient and/or operator. This accommodation may not only be an accommodation in terms of illumination level, but also various color schemes may be available and applied, e.g., in order to improve an ambient experience. Such features have shown to be helpful for reducing stress for patients and operators as well as for creating a friendly and comfortable scanning environment and atmosphere.

However, such lighting variations for the purpose of accommodation may constitute a challenge for a supervision of the medical imaging system, since modules used for such a supervision are in general configured for a supervision under certain lighting conditions. In case actual lighting conditions of a medical imaging system, e.g., for an improved ambient experience, deviate for lightening conditions the respective modules have been configured for, an accuracy and/or effectiveness of the supervision may be reduced.

### SUMMARY OF THE INVENTION

The invention provides for a method for determining one or more control parameters for controlling one or more light sources to adjust lighting conditions of a medical imaging system during an acquisition of optical sensor data using an optical sensor, for a computer program comprising machine executable instructions for determining one or more control parameters for controlling one or more light sources to adjust lighting conditions of a medical imaging system during an acquisition of optical sensor data using an optical sensor, and for a computing device for determining one or more control parameters for controlling one or more light sources to adjust lighting conditions of a medical imaging system during an acquisition of optical sensor data using an optical sensor. Furthermore, the invention provides for a medical imaging system comprising such a computing device for determining one or more control parameters for controlling one or more light sources to adjust lighting conditions of a medical imaging system during an acquisition of optical sensor data using an optical sensor.

In one aspect, the invention provides for a method for determining one or more control parameters for controlling one or more light sources to adjust lighting conditions of a medical imaging system during an acquisition of optical sensor data using an optical sensor. The optical sensor is configured for a supervision of the medical imaging system. A computing device used for executing the method comprises a target detection module, which is configured for detecting target elements of one or more target structures within optical sensor data in response to receiving the optical sensor data from the optical sensor.

The method comprises receiving the optical sensor data.

The target elements are detected within the optical sensor data using the target detection module.

In response to receiving an adjustment trigger, a feedback loop is started. The feedback loop comprises controlling an adjusting of the lighting conditions of the medical imaging system using the one or more light sources, receiving adjusted optical sensor data for the adjusted lighting conditions from the optical sensor, detecting the target elements within the adjusted optical sensor data using the target detection module, and determining confidence values for the detections of the target elements within the adjusted optical sensor data.

The feedback loop is repeated until the confidence values determined for the detections of the target elements within the adjusted optical sensor data reach a predefined confidence threshold level. One or more adjustment parameters defining the adjusting of the lighting conditions, for which the confidence values reach a predefined confidence threshold level, are determined as the one or more control parameters.

A quality of a detection of target elements of target structures within optical sensor data of a medical imaging system may depend on lighting conditions of the medical imaging system. The target detection module used for detecting the target elements is in general configured to detect target elements under specific lighting conditions. It is suggested to use a feedback loop to determine one or more adjustment parameters defining an adjusting of given lighting conditions resulting in confidence values for the detections of the target elements reaching and/or exceeding a predefined confidence threshold level. These one or more adjustment parameters are used as one or more control parameters for controlling one or more light sources to adjust lighting conditions of a medical imaging system during the acquisition of optical sensor data using the optical sensor. Thus, it may be ensured that optical sensor data is acquired under adjusted lighting conditions ensuring sufficient confidence values for the detections of the target elements.

The feedback loop and thus the adjustment of the lighting conditions may, e.g., only be executed, if necessary, i.e., in response to receiving an adjustment trigger.

Example may have the beneficial effect, that using the feedback loop adjustment parameters may be determinable for various types of given lighting conditions. Furthermore, it is not necessary to know a priori how lighting conditions for ensuring sufficient confidence values may look like, nor which parameter may have to be adjusted to ensure the sufficient confidence values. For example, using the feedback loop may enable an implementation of only a minimum adjustment for ensuring sufficient confidence values.

Examples may enable a preservation of suitable detection performance, which may be expanded using the feedback loop to a large variety of lighting preferences both in terms of light level and color. This suitable detection performance may, e.g., be a detection performance reachable in normal lighting conditions of a working environment or in any other reference lighting environment, which may have been used to configure the target detection module.

Examples may enable a stabilizing of an illumination by the light sources. This illumination may be stabilized for a supervision of the medical imaging system, in particular for a detection of target structures.

Supervision may, e.g., comprise a supervision of preparation of a patient for an examination using the medical imaging system. Using an optical sensor in combination with a target detection module, an automated or at least partially automated supervision may be implemented. Such a preparation may be time-consuming. Using an optical sensor in combination with a target detection module for an at least partial automation of the preparation may avoid drawing attention of trained and skilled operators from the patient. Thus, the operators may be enabled to better focus on the patient. Furthermore, a stress level of the operators may be reduced or kept within limits by taking over time consuming tasks, like patient preparation. This may be the case even for a high throughput, e.g., due to productivity targets of the hospital department. Thereby, limitations of consistency of examination quality, an impairing of patient and/or staff experience as well as an increase of a risk of error, including harming to the patient, may be avoided effectively.

Using an optical sensor in combination with a target detection module, assistance may be provided to an operator in the patient examination setup with a medical imaging system where needed and non-ideal used and/or setup configurations of the medical imaging system may be detected. For example, unsafe system uses and/or unsafe setup configurations may be detected. For example, a position of a patient, e.g., on a patient table, and/or positions of components of the medical imaging system, like cables and/or coils, in particular relative to the patient, may be determined.

For detecting the target elements a target detection module, which, e.g., comprises a neural network model, is used. Such a target detection module may be implemented in form of a machine learning module. For example, the target detection module may use a fully convolutional U-net architecture for detecting target elements. The U-Net architecture is described in Olaf Ronneberger, "U-Net: Convolutional Networks for Biomedical Image Segmentation" (https://arxiv.org/abs/1505.04597).

For detecting the target elements, e.g., a keypoint detection may be used. The target detection module may, e.g., be configured for a keypoint detection, i.e., a detection of keypoint elements of the target structure within the optical sensor data. Also, other methods may be used for detecting the target elements. The target detection module may, e.g., be configured for a bounding box detection, i.e., for a determination of bounding boxes identifying the target elements of the target structures. The target elements may, e.g., be part of the target structures or the target elements may be the target structures. The target detection module may, e.g., be configured for a segmentation mask detection, i.e., for a determination of segmentation masks comprising the target elements of the target structures.

For example, the target detection module may be configured to determine confidence levels for the detections of the target elements within the optical sensor data. For example, the target detection module may be configured for assigning confidence levels for the detections to the detected target elements. The target detection module may, e.g., during a training phase, be trained for assigning confidence levels to the detections to the target elements detected within the training data. These confidence levels determined using the target detection module may, e.g., be estimations trained using a loss function quantifying a deviation of the detections of the target elements from a ground truth for the target elements provided by the optical training data. The confidence levels may, e.g., be functions of an output of the target detection module. A maximum, minimum, mean or integral of the output of the detection module may be examples of quantities, which may be used to derive a confidence metric within a training distribution provided by a plurality of optical training data. Out-of-distribution confidence values may, e.g., be derived using a temporal stability of an output signal of the target detection module, a stability against perturbances of the output signal, like noise etc., or other spatial and/or temporal properties of the output signal provided by the target detection module as a response to receiving optical sensor data as input.

The predefined confidence threshold level to be reached using the feedback loop may, e.g., be a fixed value for the confidence threshold level. The feedback loop is, e.g., repeated until all the confidence levels of the target elements being detected, a mean of the confidence levels of the target elements being detected or the confidence levels of a predefined minimum number of target elements being detected reach the fixed value for the confidence threshold level. The predefined confidence threshold level to be reached by feedback loop may, e.g., be a maximum of the confidence threshold levels of the target elements being detected. For example, a mean of the confidence threshold levels of the target elements being detected is determined and the feedback loop is, e.g., repeated until the mean reaches a maximum.

The supervision may, e.g., rely on detecting target structures, like a patient's body and anatomical features and/or relevant equipment components of the medical imaging system based on optical data, like images, acquired using the optical sensor. For a reliable detection, a high precision as well as a high robustness of the detection may be needed.

In modern scanning environments of medical imaging systems, lighting may be fully accommodated to individual preferences of the patient and/or operator. This accommodation may not only be an accommodation in terms of illumination level, but also various color schemes may be available and applied, e.g., in order to improve an ambient experience. Such features have shown to be helpful for reducing stress for patient and operator as well as for creating a friendly and comfortable scanning environment and atmosphere.

However, such lighting variations for the purpose of accommodation may constitute a challenge for the target detection module, i.e., with such variations confidence values for the detections of the target elements by the target detection module may decrease. The target detection module may be configured for detecting target elements under certain basic lighting conditions. However, these basic lighting conditions may often be unknown or it may be unknown, which features comprised by the lighting conditions are most influential for the quality of detection of the target elements. In particular, in case of a trained target detection module, trained to detect target elements using machine learning, the precise basic lighting conditions underlying the learning may be unknown. For the detection of the target elements, e.g., a keypoint element detection, i.e., a detection of keypoint features of the target structures, a bounding box detection, a segmentation mask detection, etc. may be used.

Even in case the basic lighting conditions are known, it may be challenging to match these basic lighting conditions for a real environment of a medical imaging system or it may be sufficient to only adjust individual features of the lighting conditions, in order to achieve a sufficient level of confidence for the detections of the target elements.

For example, multiple light sources may be implemented in form of a directional color light array comprising the light sources. The light sources may, e.g., be provided by a set of light emitting diodes (LEDs) and/or lasers. These light sources may, e.g., comprise light sources of basic colors, like red, green and blue. For example, the light sources may be implemented in form of a projector, e.g., LED or laser. For example, the light sources may be implemented in form of a combination of a directional color light array and a projector.

For example, a low additional lighting level may be sufficient for ensuring confidence levels reaching the predefined confidence threshold level. This may, e.g., be achieved by directional characteristics of the additional illumination, a flexible choice of color settings of the additional illumination and/or the fact that the additional illumination may be needed in low-light settings. For example, if an ambient light preference is for low blue light illumination, only a low and local addition of green and/or red light may be needed for a sufficient detection responses, i.e., to achieve confidence levels reaching the predefined confidence threshold level.

Using a feedback loop may enable a selective restoration of predefined detection responses, i.e., confidence levels reaching a predefined threshold level, e.g., only when a deviation from a predefined behavior is detected. Such a deviation may, e.g., have the form of a low and/or noisy response resulting in confidence levels below the predefined threshold level. For example, if low output activation levels of the target detection module, e.g., comprising a neural network, are detected, an optimizer, e.g., a constrained optimizer, may be used for adjusting lighting conditions until predefined threshold is reach. Alternatively, the lighting conditions may be adjusted, until a maximum activation, i.e., a maximum for the confidence levels, is reached.

Using a feedback loop may have the beneficial effect, that no a priori knowledge of the current lighting conditions, of adjustments of the lighting conditions to be applied, and/or of the lighting conditions to be achieved by the respective adjustments is required.

Alternatively, direct metrics determined using raw optical sensor data camera and/or processed optical sensor data, like optical images, may be used to determine, whether an adjustment of the lighting conditions is required. Such direct metrics may, e.g., comprise a noise level, a light level, a gain and/or exposure settings.

In general, it may, e.g., be assumed that a total dropout of detection response is rather unlikely, even in case of almost completely dark environments. Thus, examples may make use of low duty cycles for the additional illumination, which may also be synchronized with the actual exposure of the optical sensor.

For example, even in case of no dedicated illumination of the medical imaging system, in most cases there will at least be some residual indirect illumination from light sources, like monitors, control panels, control lights, etc. Thus, even without dedicated illumination a detection response may to be expected.

According to another example, the lighting may, e.g., smoothly and continuously be adjusted at a different, e.g., lower rate than a frame rate of optical images provided using the optical sensor for a visualization of the medical imaging system for a human observer, e.g., for an operator,, until a relevant detection of target elements is completed and may then be turned off again. Such an approach may have the beneficial effect of reducing irritations, e.g., of a patient and/or an operator of the medical imaging system light, due to lighting adjustment, e.g., additional lighting, from the one or more light sources controlled to the adjust lighting conditions.

In another example, the light may, e.g., be modulated at a higher rate than human perception and/or a frame rate optical images provided using of the optical sensor for a visualization of the medical imaging system for a human observer, e.g., for an operator. It may, e.g., optionally be synched with an exposure rate and/or frame rate of the optical sensor. Such an approach may have the beneficial effects of keeping the duty-cycle low, of reducing interferences with ambient light settings and/or of avoiding possible irritations due to a perceivable flickering.

A directional color light array may, e.g., be implemented using an LED ring array of alternating base colors surrounding an optical sensor, e.g., a lens of the optical sensor, such that illumination shadows from the additional light emitted by the directional color light array may be restricted. The ring array may be arranged concentric with respect to the optical sensor and/or other optics. For example, the color of different ring sections may be controlled individually. Such an approach may increase a controllability of the lighting adjustments in terms of light level and/or color. In addition, individually controllable ring sections may further allow for a plurality of different status indications identifying different states of the optical sensor and/or of the medical imaging system. The status indications may, e.g., comprise a dynamic behavior of the array in sub-regions of the array, e.g., of individual ring sections, and/or the array in total.

For example, neither any training of the target detection module has to be adjusted or repeated for specific lighting conditions applied for a medical imaging system, nor do the lighting conditions of the medical imaging system have to be adjusted to precisely match the training conditions, under which the target detection module has been trained.

For example, an increase of network size and computational effort as well as an increase of training datasets for training such networks may thereby be prevented.

The optical sensor may, e.g., be a monochrome sensor configured to acquire optical sensor data of a single wavelength or sensor data within a single, in particular narrow, range of wavelengths corresponding to a single color. For example, wavelengths corresponding to the color violet are comprised by the range of wavelengths from 380 nm to 450 nm, wavelengths corresponding to the color blue are comprised by the range of wavelengths from 450 nm to 485 nm, wavelengths corresponding to the color cyan are comprised by the range of wavelengths from 485 nm to 500 nm, wavelengths corresponding to the color green are comprised by the range of wavelengths from 500 nm to 565 nm, wavelengths corresponding to the color yellow are comprised by the range of wavelengths from 565 nm to 590 nm, wavelengths corresponding to the color orange are comprised by the range of wavelengths from 590 nm to 625 nm, and wavelengths corresponding to the color red are comprised by the range of wavelengths from 625 nm to 750 nm.

For example, the color of light acquired by the monochrome optical sensor may be invisible for the human eye, e.g., within the infrared (IR) range and in particular within the near infrared (NIR). Wavelengths corresponding to the NIR range are comprised by the range from 750 nm to 1400 nm, i.e., 1.4 µm. For example, wavelength of 10 µm at room temperature may be used.

The one or more light sources may, e.g., be monochrome light sources configured to emit light of the same single wavelength or light within the same single, in particular narrow, range of wavelengths corresponding to a single color. For example, the color of light emitted by the monochrome light sources may be invisible for the human eye, e.g., within the infrared (IR) range and in particular within the near infrared (NIR).

Using light of wavelengths invisible human eye may, e.g., reduce an interfering of the of the lighting with the ambient lighting preferences and vice versa.

For example, the color of light acquired by the monochrome optical sensor may be a polychrome optical sensor configured to acquire light of different wavelengths corresponding to a plurality of colors. The one or more light sources may, e.g., comprise one or more polychrome light sources configured to emit light of a plurality of different wavelengths and/or a combination of a plurality of different monochrome light sources, which are configured to emit light of different wavelengths.

Using polychrome light for illuminating the scene of the medical imaging system, within which targets are to be detected using the target detecting module, may have the beneficial effect that different color channel, i.e., wavelength dimension may be used to improve the detection. Different wavelength dimensions may, e.g., be beneficial for improving a number of exploitable features for the respective detections of targets. For example, a distinction of patients and/or objects, like components of the medical imaging system, may be improved. Thus, respective detection failure rates, like false negatives and/or false positives, may be reduced compare to detections using only a single wavelength dimension, i.e., light of a single color.

In general, normal lighting settings may represent a statistically likely clinical usage scenario which can be expected for many standard examinations using a medical imaging system, so that an overall performance may statistically be significantly determined by the best hardware and algorithm choice for these cases.

For example, low light conditions may be used for operating the medical imaging system. Besides creating a comfortable environment, low light conditions may, e.g., also be beneficial for improving visibility of displays of instruments and equipment, e.g., displays of the medical imaging system, on one hand and for providing an additional illumination of the examination target and/or patient area on the other hand. Such an additional illumination may be target-oriented and/or context-dependent. It may be adapted to individual patients, operators and/or work steps.

A plurality of light sources may, e.g., be provided in form a directional color light array. The directional color light array may, e.g., be attached to and/or configured in accordance with the optical sensor. Elements of the directional color light array may, e.g., be flexibly and individually controllable in terms of light level and/or color. A directional color light array may be used to compensating for a lack of illumination in a target area, in particular color-specific illumination features.

For example, an algorithm and feedback loop may be provided for detecting current lighting conditions of the medical imaging system, e.g., of a room housing the medical imaging system, determining a detection response by the target detection module and controlling the one or more light sources such that the target elements to be detected, e.g., a patient's body and/or relevant objects, like components of the medical imaging system, may be illuminated independently from and not interfering with ambient lighting conditions. The illumination may be varied based on a detection response level using, e.g., a constrained optimizer, like a gradient optimizer. Constraints may, e.g., be used to ensure eye safety and comfort of a patient and/or operator.

For example, an algorithm may be provided for controlling the one or more light sources such that patient's eyes are spared and/or that an adjustment of the illumination, e.g., comprising an additional illumination level, is only provided when needed. For example, the adjustment of the illumination may only be provided for individual frames.

For example, an algorithm may be provided for controlling the one or more light sources such that users, like patients and/or operators, may be enable to immediately understand a system status and/or which functions are currently used and/or performed, i.e., for providing a prominent and well-visible, flexible and detailed status indication of the optical sensor and/or the medical imaging system.

For example, the adjustment trigger comprises determining confidence values for the detections of the target elements within the optical sensor data below the predefined confidence threshold level.

In case it is determined that target elements are determined with confidence values below the predefined confidence threshold level, i.e., that a quality of the detections of target elements is insufficient, the feedback loop is triggered to improve the quality of the detections of target elements using an adjustment of the lighting conditions of the medical imaging system. For example, the adjustment trigger comprises determining a predefined number of confidence values below the predefined confidence threshold level.

For example, the confidence values below the predefined confidence threshold level are determined for the detections of the target elements within one or more spatial sections of interest within the optical sensor data. Example may have the beneficial effect that an improvement of the quality of the detections of target elements within one or more spatial sections of interest may be ensured using an adjustment of the lighting conditions of the medical imaging system. Thus, the adjusting if the lighting conditions may be focused on spatial sections of interest. Preferably the spatial sections of interest are spatial section comprising target elements to be detected and/or spatial section expecting to comprise target elements to be detected.

For example, an additional control module, e.g., an AI-controlled type of control module like, e.g., a machine learning module, may be used to control in dependence of a context and/or a procedure step an illuminate of relevant areas of a scene detected by the additional control module using the optical sensor. For illumination the relevant areas determined by the additional control module, the determined one or more control parameters may be used. The additional control module may, e.g., use the target elements detected using the target detection module to determine the relevant areas to be illuminated.

For example, the method further comprises determining quality parameters for the received optical sensor data. The adjustment trigger comprises detecting one or more of the following characteristics of the determined quality parameters: a signal-to-noise level below a predefined signal-to-noise level threshold, a total light intensity level below a predefined total light intensity threshold, a color-specific light intensity level below a predefined color-specific light intensity threshold, an intensity difference between different colors of light exceeding a predefined color intensity difference threshold.

For example, a requirement to improve the quality of the detection of the target elements may be indicated by a detection of one or more insufficient quality parameters for the received optical sensor data. A detection of one or more insufficient quality parameters may be used as an adjustment trigger triggering an improvement of the quality of the detection of the target elements.

For example, a requirement to improve the quality of the detection of the target elements is indicated by a signal-to-noise level below a predefined signal-to-noise level threshold. A detection of such a signal-to-noise level below a predefined signal-to-noise level threshold may thus be used as an adjustment trigger for triggering the feedback loop for adjusting the lighting conditions.

For example, a requirement to improve the quality of the detection of the target elements is indicated by a total light intensity level below a predefined total light intensity threshold. A detection of such a total light intensity level below a predefined total light intensity threshold may thus be used as an adjustment trigger for triggering the feedback loop for adjusting the lighting conditions.

For example, a requirement to improve the quality of the detection of the target elements is indicated by a color-specific light intensity level below a predefined color-specific light intensity threshold. A detection of such a color-specific light intensity level below a predefined color-specific light intensity threshold may thus be used as an adjustment trigger for triggering the feedback loop for adjusting the lighting conditions.

The color-specific light intensity level may be a light intensity level of a single color of light or of a specific combination of colors of light. For example, the color-specific light intensity level may describe an absolute intensity level. For example, the color-specific light intensity level may describe relative intensity level. For describing relative color-specific light intensity levels, vector is color space, e.g., RGB space, may be used. A predefined color-specific light intensity threshold may, e.g., be described by an angle between a given first vector in color space describing a given color-specific light intensity level and a second reference vector in color space. In case the deviation of the first vector from the second reference vector, i.e., the angle between the two vectors is larger than a threshold angle, the color-specific light intensity level is considered to be below the predefined color-specific light intensity threshold. The second reference vector may, e.g., be a mean reference vector.

For example, a reference vector c = [0.4, 0.92, 0.0] in RGB space, e.g., in a region of interest, may be given. The reference vector c may, e.g., be a mean vector of color vectors in the region of interest. For example, a vector c' = [0.707, 0.707, 0.0] is measured describing a relative color-specific light intensity level. Then, a = acos (c * c') describes the angle between the two color vectors, i.e., how large a deviation of measured vector c' from reference vector c is. In case the color-specific light intensity level described c' = [0.707, 0.707, 0.0] is below a predefined color-specific light intensity threshold, i.e., the angle α is larger than a predefined threshold angle, an adjustment trigger may be given triggering the feedback loop.

For example, a requirement to improve the quality of the detection of the target elements is indicated by an intensity difference between different colors of light exceeding a predefined color intensity difference threshold. A detection of such an intensity difference between different colors of light exceeding a predefined color intensity difference threshold may thus be used as an adjustment trigger for triggering the feedback loop for adjusting the lighting conditions.

For example, the quality parameters are determined for one or more spatial sections of interest within the optical sensor data. Example may have the beneficial effect that an on-demand improvement of the quality of the detections of target elements is triggered by a detection of insufficient quality parameters for one or more spatial sections of interest. Thus, a monitoring of quality parameter may be focused on spatial sections of interest ensuring. Preferably the spatial sections of interest are spatial section comprising target elements to be detected and/or spatial section expecting to comprise target elements to be detected.

For example, the adjusting of the lighting conditions comprises one or more of the following: an increasing of a level of a total light intensity of an illumination by one or more of the light sources, an increasing of a color-specific light intensity of the illumination by one or more of the light sources, a shifting of a region of illumination by one or more of the light sources spatially.

For example, the adjusting of the lighting conditions during the feedback loop comprises an increasing of the level of the total light intensity of the illumination by one or more of the light sources.

For example, the adjusting of the lighting conditions during the feedback loop comprises an increasing of the color-specific light intensity of the illumination by one or more of the light sources.

For example, the adjusting of the lighting conditions during the feedback loop comprises a spatial shifting of the region of illumination by one or more of the light sources.

For example, the method further comprises applying one or more constraints to the adjusting of the lighting conditions. The one or more constraints comprise one or more of the following: an exclusion of one or more spatial sections within the optical sensor data from the illumination by one or more of the light sources, a predefined maximum total light intensity threshold for the total light intensity of the illumination by one or more of the light sources, a predefined maximum color-specific light intensity threshold for a color-specific light intensity of the illumination by one or more of the light sources.

Examples may have the beneficial effect that by applying constraints of the adjusting of the lighting conditions impairments of a patient present within the medical imaging system may be avoided. For example, interferences with an ambient lighting of the medical imaging system may be avoided or at least be limited.

For example, the constraints comprise an exclusion of one or more spatial sections within the optical sensor data from the illumination by one or more of the light sources. Spatial sections being excluded may, e.g., comprise a head of a patient, in particular eyes of the patient.

For example, the constraints comprise a predefined maximum total light intensity threshold for the total light intensity of the illumination by one or more of the light sources.

For example, the constraints comprise a predefined maximum color-specific light intensity threshold for a color-specific light intensity of the illumination by one or more of the light sources.

For example, an adjustment rate of the adjusting of the lighting conditions is lower than a predefined frame rate of optical images, which are provided using the optical sensor data received from the optical sensor. By slowly adjusting the lighting conditions irritations of the patient and/or operator of the medical imaging system may be avoided. In particular, sudden changes of the lighting conditions, like a flickering, may be avoided.

For example, an adjustment rate of the adjusting of the lighting conditions is higher than the predefined frame rate of optical images. For example, adjustments may be executed faster than perceptible by a patient and/or an operator. Thus, irritations of the patient and/or operator of the medical imaging system may be avoided or reduced. For example, the adjustments may be executed in intervals. These intervals may be shorter than the inverse frame rate of optical images, while pauses between these intervals may be longer than the inverse frame rate of optical images.

For example, only a few adjustments may be required, which can be tested within a single interval of time shorter than the inverse frame rate of optical images. If more adjustments may be necessary, they may be executed within one or more additional intervals of time shorter than the inverse frame rate of optical images.

For example, the adjustment rate is higher than 30 Hz. For example, the adjustment rate is higher than 60 Hz. For example, the adjustment rate is 90Hz, 120Hz, 150Hz, 180Hz or higher.

For example, the method further comprises using the determined one or more control parameters for controlling the light sources for the supervision of the medical imaging system using the optical sensor. By controlling the light sources using the one or more control parameters determined, lighting conditions for the supervision of the medical imaging system may be improved, such that confidence values of the detections of the target elements may reach and/or exceed the predefined confidence threshold level.

For example, the light sources are controlled to illuminate the medical imaging system continuously using the one or more control parameters independent of optical sensor data acquisition by the optical sensor. Illuminating the medical imaging system continuously using the one or more control parameters independent of optical sensor data acquisition by the optical sensor may have the beneficial effect, that the illumination is not altered in case of optical sensor data acquisition by the optical sensor, since the illumination is already adjusted for the acquisition. Thus, irritations of the patient and/or the operator of the medical imaging system due to changes, in particular due to sudden chances causing a flickering of the illumination, may be avoided.

For example, the light sources are controlled to synchronize the illumination of the medical imaging system with the light sources using the one or more control parameters with the optical sensor data acquisition by the optical sensor. Example may have the beneficial effect, that the illumination may only be altered using the control parameters, when needed, i.e., when optical sensor data is acquired. Thus, the illumination may, e.g., only be altered for acquiring single frames, i.e., images. By using the adjusted illumination for a short period of time only, e.g., faster than human perception, the adjustment may not be perceptible or may at least not be irritating for the patient and/or for the operator of the medical imaging system.

Furthermore, the one or more light sources may be configured for emitting a status light indicating a status. For example, a status of the one or more light sources or a status of the medical imaging system may be indicated.

Examples may provide clear and well visible information about a system activation status for a variety of features and functions.

Examples may have the beneficial effect of supporting a controllability of the usage of the optical sensor for the supervision of the medical imaging system in accordance with privacy regulation needs of both patients and operators. For example, an acceptance of introducing such systems may be improved by making the system activity and/or status visible so that patients and/or operators may be reminded of the optical sensor and may opt to deactivate the system, whenever preferred. Such systems may, e.g., be based on scalable sensor and compute platforms and may be designed to support a large set of potential functions and features, which may be configured by software options depending on the user's preferences. System status may therefore not just a binary value, but multiple different states of the system may be indicated by a plurality of different status lights. For example, different combinations of colors of light emitted as status light may indicate different states of the system. For example, the status light may be blinking light. Different frequencies of blinking or different combinations of blinking, e.g., for different colors of light, emitted as status light may indicate different states of the system.

The status indication feature may, e.g., be independent from the ambient light settings and resulting detection response. It may, e.g., use a base illumination level for the directional light array so that the user may always can see the indication indicating the system's current status and/or a function being currently performed. For improved visibility in bright ambient light settings also the status indication can be controlled via a feedback loop from the optical sensor.

A directional color light array may, e.g., be implemented using an LED ring array of alternating base colors surrounding an optical sensor, e.g., a lens of the optical sensor, such that illumination shadows from the additional light emitted by the directional color light array may be restricted. The ring array may be arranged concentric with respect to the optical sensor and/or other optics. For example, the color of different ring sections may be controlled individually. Such an approach may increase a controllability of the lighting adjustments in terms of light level and/or color. In addition, individually controllable ring sections may further allow for a plurality of different status indications identifying different states of the optical sensor and/or of the medical imaging system. The status indications may, e.g., comprise a dynamic behavior of the array in sub-regions of the array, e.g., of individual ring sections, and/or the array in total.

In another aspect, the invention provides for a computer program comprising machine executable instructions for determining one or more control parameters for controlling one or more light sources to adjust lighting conditions of a medical imaging system during an acquisition of optical sensor data using an optical sensor. The optical sensor is configured for a supervision of the medical imaging system. A target detection module is configured for detecting target elements of one or more target structures within optical sensor data in response to receiving the optical sensor data from the optical sensor.

Execution of the machine executable instructions by a processor of a computing device causing the processor to control the computing device to execute a method for the determining the one or more control parameters for controlling the one or more light sources.

The method comprises receiving the optical sensor data.

The target elements are detected within the optical sensor data using the target detection module.

In response to receiving an adjustment trigger, a feedback loop is started. The feedback loop comprises controlling an adjusting of the lighting conditions of the medical imaging system using the one or more light sources, receiving adjusted optical sensor data for the adjusted lighting conditions from the optical sensor, detecting the target elements within the adjusted optical sensor data using the target detection module, and determining confidence values for the detections of the target elements within the adjusted optical sensor data.

The feedback loop is repeated until the confidence values determined for the detections of the target elements within the adjusted optical sensor data reach a predefined confidence threshold level. One or more adjustment parameters defining the adjusting of the lighting conditions, for which the confidence values reach a predefined confidence threshold level, are determined as the one or more control parameters.

For example, the machine executable instructions may be configured for causing a computing device to executing any of the aforementioned examples of a method for determining one or more control parameters for controlling one or more light sources.

In another aspect, the invention provides for a computing device for determining one or more control parameters for controlling one or more light sources to adjust lighting conditions of a medical imaging system during an acquisition of optical sensor data using an optical sensor. The optical sensor is configured for a supervision of the medical imaging system. The computing device comprises a target detection module, which is configured for detecting target elements of one or more target structures within optical sensor data in response to receiving the optical sensor data from the optical sensor.

The computing device comprises a processor and a memory with machine executable instructions stored therein. Execution of the machine executable instructions by the processor of the computing device causing the processor to control the computing device to execute a method for the determining the one or more control parameters for controlling the one or more light sources.

The method comprises receiving the optical sensor data.

The target elements are detected within the optical sensor data using the target detection module.

In response to receiving an adjustment trigger, a feedback loop is started. The feedback loop comprises controlling an adjusting of the lighting conditions of the medical imaging system using the one or more light sources, receiving adjusted optical sensor data for the adjusted lighting conditions from the optical sensor, detecting the target elements within the adjusted optical sensor data using the target detection module, and determining confidence values for the detections of the target elements within the adjusted optical sensor data.

The feedback loop is repeated until the confidence values determined for the detections of the target elements within the adjusted optical sensor data reach a predefined confidence threshold level. One or more adjustment parameters defining the adjusting of the lighting conditions, for which the confidence values reach a predefined confidence threshold level, are determined as the one or more control parameters.

For example, the computing device may be configured for executing any of the aforementioned examples of a method for determining one or more control parameters for controlling one or more light sources.

In another aspect, the invention provides for a medical imaging system. The medical imaging system comprises any of the aforementioned examples of the computing device, one or more light sources configured for illuminating the medical imaging system, and an optical sensor configured for a supervision of the medical imaging system. The medical imaging system is one of the following: a magnetic resonance imaging system, a computed tomography system, an X-ray imaging system.

The technology described herein may be applied to the use of optical sensors in various medical applications, i.e., for various medical imaging systems. The medical imaging system, like e.g., an MRI system, may be comprised by a radiation therapy (TR) system. The X-ray imaging system may, e.g., be part of an image guided therapy (IGT) system. The X-ray imaging system may, e.g., be a diagnostic X-ray (DXR) system.

It is understood that one or more of the aforementioned embodiments of the invention may be combined as long as the combined embodiments are not mutually exclusive.

As will be appreciated by one skilled in the art, aspects of the present invention may be embodied as an apparatus, method, or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

The term `machine learning' (ML) refers to a computer algorithm used to extract useful information from training datasets by building probabilistic models, which are referred to as machine learning modules or models, in an automated way. A machine learning module may also be referred to as a predictive model. Machine learning algorithms build a mathematical model based on sample data, known as `training data', in order to make predictions or decisions without being explicitly programmed to perform the respective task. The machine learning module may be performed using a learning algorithm such as supervised or unsupervised learning. The machine learning module may be based on various techniques such as clustering, classification, linear regression, reinforcement, self-learning, support vector machines, neural networks, etc. A machine learning module may, e.g., be a data structure or program such as a neural network, in particular a convolutional neural network, a support vector machine, a decision tree, a Bayesian network etc. The machine learning module may be adapted, i.e., trained to predict an unmeasured value. The trained machine learning module may thus be enabled to predict the unmeasured value as output from other known values as input.

A machine learning module to be trained may, e.g., be an untrained machine learning module, a pre-trained machine learning module or a partially trained machine learning module. The machine learning module being trained may be an untrained machine learning module, which is trained from scratch. Alternatively, the machine learning module being trained may be a pre-trained or partially trained machine learning module. In general, it may not be necessary to start with an untrained machine learning module, e.g., in deep learning. For example, one may start with a pre-trained or partially trained machine learning module. The pre-trained or partially trained machine learning module may have been pre-trained or partially trained for the same or a similar task. Using a pre-trained or partially trained machine learning may, e.g., enable a faster training of the trained machine learning module to be trained, i.e., the training may converge faster. For example, transfer learning may be used for training a pre-trained or partially trained machine learning module. Transfer learning refers to a machine learning process, which rather than starting the learning process from scratch starts from patterns that have been previously learned, when solving a different problem. This way previous learnings may, e.g., be leveraged, avoiding to start from scratch. A pre-trained machine learning module is a machine learning module that was trained previously, e.g., on a large benchmark dataset to solve a problem similar to the one to be solved by the additional learning. In case of a pre-trained machine learning module a previous learning process has been completed successfully. A partially trained machine learning module is a machine learning module, which has been partially trained, i.e., the training process may not have been completed yet. A pre-trained or partially machine learning module may, e.g., be import and trained to be used for the purposes disclosed herein.

### BRIEF DESCRIPTION OF THE DRAWINGS

In the following preferred embodiments of the invention will be described, by way of example only, and with reference to the drawings in which:
Fig. 1 illustrates an exemplary method for determining one or more control parameters for controlling one or more light sources;
Fig. 2 illustrates an exemplary optical sensor system comprising a plurality of light sources;
Fig. 3 illustrates an exemplary medical imaging system environment without adjustments of the lighting conditions;
Fig. 4 illustrates the exemplary medical imaging system environment of Fig. 3 with adjustments of the lighting conditions;
Fig. 5 illustrates an exemplary computing device for determining one or more control parameters for controlling one or more light sources;
Fig. 6 illustrates an exemplary medical imaging system comprising a computing device;
Fig. 7 illustrates exemplary optical sensor training data for training a target detection module;
Fig. 8 illustrates an exemplary method for training a target detection module;
Fig. 9 illustrates an exemplary detection of target elements in optical sensor data;
Fig. 10 illustrates another exemplary detection of target elements in optical sensor data; and
Fig. 11 illustrates another exemplary detection of target elements in optical sensor data.

### DETAILED DESCRIPTION OF EMBODIMENTS

Like numbered elements in these figures are either equivalent elements or perform the same function. Elements which have been discussed previously will not necessarily be discussed in later figures if the function is equivalent.

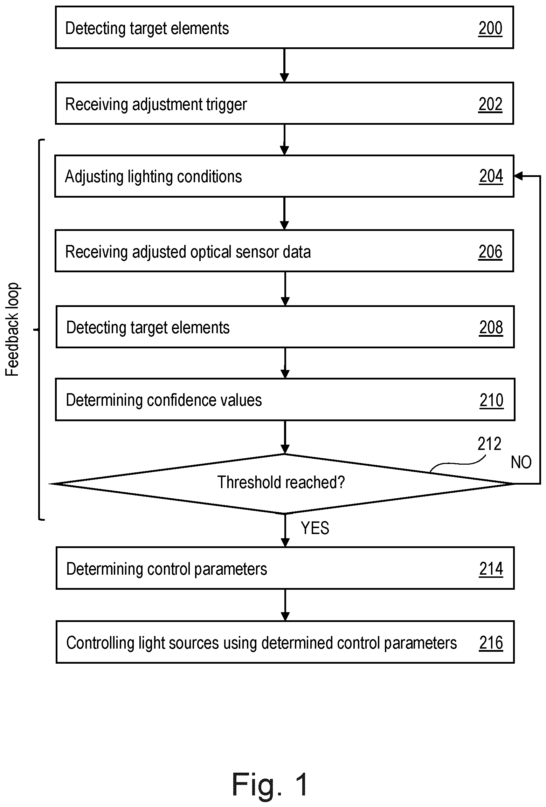

Fig. 1 illustrates an exemplary method for determining one or more control parameters for controlling one or more light sources to adjust lighting conditions of a medical imaging system during an acquisition of optical sensor data using an optical sensor. The optical sensor is configured for a supervision of the medical imaging system. A computing device used for executing the method comprises a target detection module, which is configured for detecting target elements of one or more target structures within optical sensor data in response to receiving the optical sensor data from the optical sensor.

In block 200, the target elements are detected within the optical sensor data using the target detection module.

In block 202, an adjustment trigger is received. In response to receiving an adjustment trigger, a feedback loop is started. The feedback loop comprises blocks 204 to 212. In block 204, an adjusting of the lighting conditions of the medical imaging system using the one or more light sources is controlled. In block 206, adjusted optical sensor data for the adjusted lighting conditions from the optical sensor is received. In block 208, the target elements within the adjusted optical sensor data are detected using the target detection module. In block 210, confidence values for the detections of the target elements within the adjusted optical sensor data are determined. In block 212, the confidence values determined for the detections of the target elements within the adjusted optical sensor data are checked.

In case the confidence values determined for the detections of the target elements within the adjusted optical sensor data do not reach a predefined confidence threshold level, the method continues with block 204 and the feedback loop is repeated. The feedback loop is repeated until the confidence values determined for the detections of the target elements within the adjusted optical sensor data reach a predefined confidence threshold level.

In case the confidence values determined for the detections of the target elements within the adjusted optical sensor data reach a predefined confidence threshold level, the method continues with block 214. In block 214, one or more adjustment parameters defining the adjusting of the lighting conditions, for which the confidence values reach a predefined confidence threshold level, are determined as the one or more control parameters.

In block 216, the determined one or more control parameters ate used for controlling the light sources for the supervision of the medical imaging system using the optical sensor.

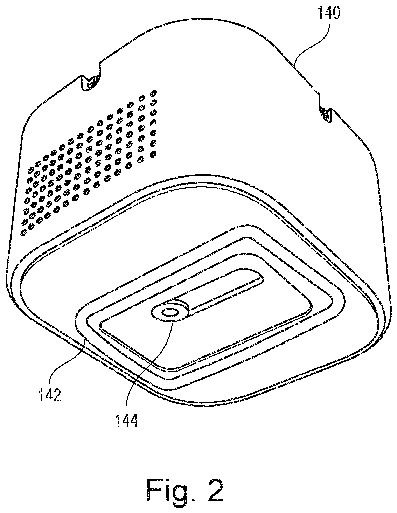

Fig. 2 illustrates exemplary light sources 142. The light sources 142 are integrated into an optical sensor system 140. In addition to the light sources 142, the optical sensor system 140 comprises at least one optical sensor 144, e.g., a camera. The light sources 142 are provided in form of a directional color light array. The light array comprises a plurality of elements, i.e., light sources 142, which may be flexibly controlled individually, e.g., in terms of light level and color. Such a directional color light array may be used for compensating a lack of illumination in a target area.

The directional color light array with the light sources 142 may, e.g., be implemented using an LED ring array of alternating base colors surrounding the optical sensor 144, e.g., a lens of the optical sensor 144, as shown in Fig. 2. Thus, illumination shadows from the additional light emitted by the directional color light array with the light sources 142 may be restricted. The ring array may be arranged concentric with respect to the optical sensor 144 and/or other optics. For example, the color of different ring sections may be controlled individually. Such an approach may increase a controllability of the lighting adjustments in terms of light level and/or color. In addition, individually controllable ring sections may further allow for a plurality of different status indications identifying different states of the optical sensor system 144 and/or of the medical imaging system. The status indications may, e.g., comprise a dynamic behavior of the array in sub-regions of the array, e.g., of individual ring sections, and/or the array in total.

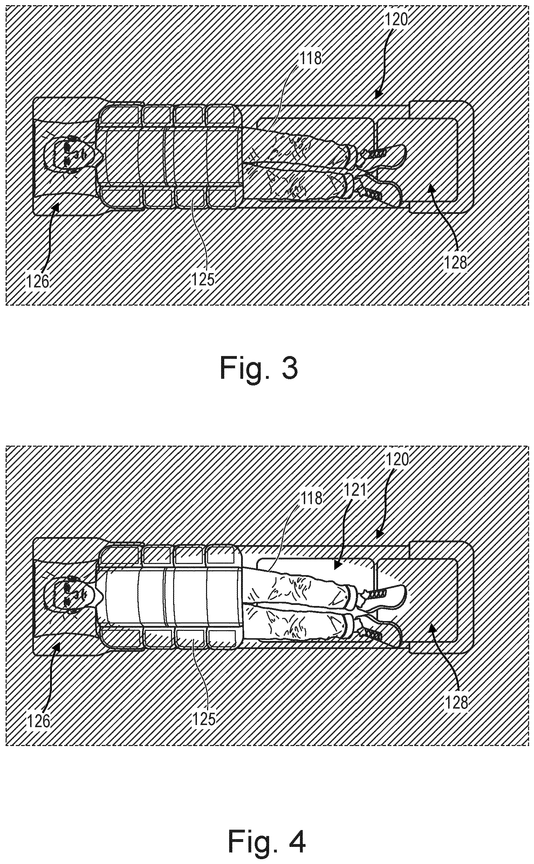

Fig. 3 illustrates an exemplary medical imaging system environment without adjustments of the lighting conditions. Shown is patient table 120 of a medical imaging system, e.g., a magnetic resonance system. An upper surface 126 of the patient table 120 comprises a relining surface 128, on which a patient 118 is lying. Over the patient 118 is an anterior coil 125 of the magnetic resonance imaging system arranged. For example, an ambient illumination may be dimmed, in order to provide a relaxing atmosphere for the patient 118. However, the low ambient illumination may cause problems for a target detection module to detect target elements of one or more target structures. The target elements to be detected may, e.g., be target elements of the ambient coil 125, of the patient 118, and/or of the patient table 120.

Fig. 4 illustrates the exemplary medical imaging system environment of Fig. 3 with adjustments of the lighting conditions. The adjustment of the lighting conditions comprises an additional illumination in a target area 121, e.g., the anterior coil of the magnetic resonance imaging system. Within the target area 121, e.g., a total lighting level is increased. For example, a constraint is applied to the adjustment of the lighting conditions in the form that a head of the patient 118 is excluded from the additional illumination, in order to prevent a blinding of the patient 118. Such constraints may be used to ensure eye safety and/or comfort of a patient. In the exemplary case illustrated in Fig. 4, illumination in the target area 121, i.e., the anterior coil 125 is improved. For example, the illumination may be improved during an acquisition of optical sensor data for a single frame using the optical sensor. In order to determine adjustment parameters defining a sufficient adjustment of the lighting conditions, the illumination in the target area 121 may, e.g., be modified until a sufficient detection response is reached, i.e., until confidence values of target elements detected within the target area 121 reach a predefined threshold. For the modification, a feedback loop may be used and repeated as described for the method of Fig. 1, until the confidence values of the target elements detected within the target area 121 reach the predefined threshold. The resulting adjustment parameters may be used as control parameters for controlling one or more light sources to illuminate the target area 121 as shown in Fig. 4. This additional illumination of the patient body 118 and other relevant objects 125 within the target area 121 is independent from and/or not interfering with the ambient lighting conditions.

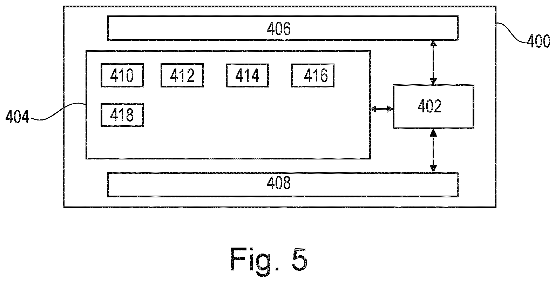

Fig. 5 illustrates an exemplary computing device 400 for determining one or more control parameters for controlling one or more light sources. The computing device 400 is shown as comprising a computational component 402. The computational component 402 is intended to represent one or more processors or processing cores or other computational elements. The computational component 402 is shown as being connected to a hardware interface 406 and a memory 404. The hardware interface 406 may enable the computational component 402 to exchange commands and data with other components, e.g., one or more lighting sources and/or an optical sensor. The hardware interface 406 may for example enable the computational component 402 to control the one or more lighting sources to adjust lighting conditions of a medical imaging system at least during an acquisition of optical sensor data using the optical sensor. The hardware interface 406 may for example further enable the computational component 402 to control the optical sensor to acquire the optical sensor data. The hardware interface 406 may for example further enable the computational component 402 to control a medical imaging system.

The computational system 404 is further shown as being connected to a user interface 408 which may for example enable an operator to control and operate the computing device 400 and via the computing device 400 one or more light sources, an optical sensor, and/or a medical imaging system. The user interface 408 may, e.g., comprise an output and/or input device enabling a user to interact with the computer 400. The output device may, e.g., comprise a display device configured for displaying magnetic resonance images 426. The input device may, e.g., comprise a keyboard and/or a mouse enabling the user to insert control commands for controlling the computing device 400 and via the computing device 400 the one or more light sources, the optical sensor, and/or the medical imaging system.

The memory 404 is shown as containing machine-executable instructions 410. The machine-executable instructions 410 enable the computational component 402 to perform controlling tasks, such as controlling the one or more lighting sources and/or the optical sensor, to perform numerical tasks, as well as performing various signal data processing tasks. The machine-executable instructions 410 may, e.g., enable the computational component 402 and thus the computing device 400 to determining one or more control parameters for controlling the one or more light sources to adjust the lighting conditions of the medical imaging system during the acquisition of optical sensor data using the optical sensor according to the method of Fig. 1. For example, the instructions 410 may, e.g., enable the computational component 402 and thus the computing device 400 to control a medical imaging system.

The memory 404 is further shown as containing a target detection module 412. The target detection module 412 is configured for detecting target elements of one or more target structures within optical sensor data 414 in response to receiving the optical sensor data 414 from the optical sensor.

The memory 404 is further shown as containing optical sensor data 414. The optical sensor data 414 has been acquired using an optical sensor configured for a supervision of a medical imaging system.

The memory 404 is further shown as containing one or more control parameters 416 for controlling one or more light sources to adjust lighting conditions of the medical imaging system during the acquisition of optical sensor data using the optical sensor.

The memory 404 may optionally further contain training data 418, e.g., synthetic training data, configured for training the target detection module 412 to detect target elements of one or more target structures within the optical sensor data 414 in response to receiving the optical sensor data 414 from the optical sensor.

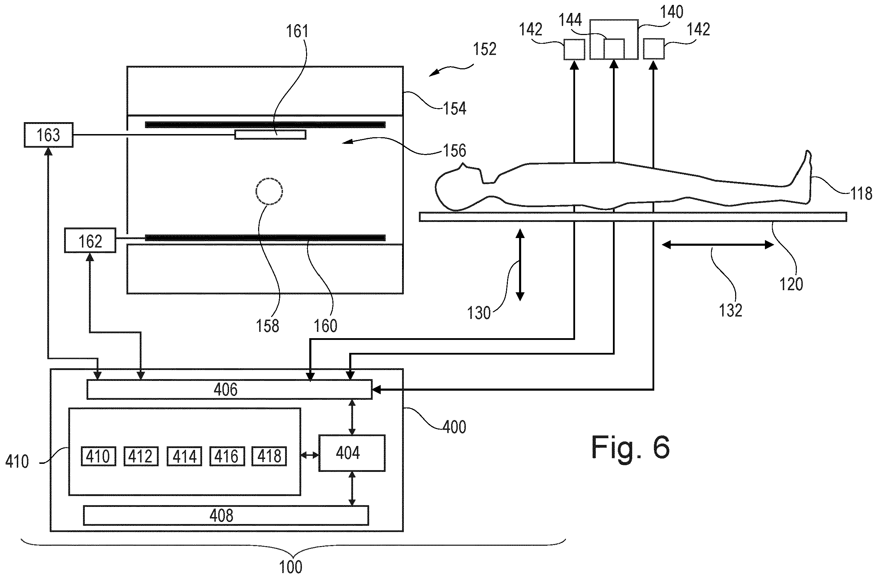

Fig. 6 an exemplary medical imaging system 100 comprising a computing device 400. The computing device 400 is configured for determining one or more control parameters for controlling one or more light sources 412. The computing device 400 of Fig. 6 is, e.g., the computing device 400 of Fig. 5.

The medical imaging system 100, e.g., comprises one or more light sources 142 configured for illuminating the medical imaging system 100 and an optical sensor 144 configured for a supervision of the medical imaging system 100. The optical sensor 144 is, e.g., provided by an optical sensor system 140. The optical sensor system 140 may, e.g., be implemented in form of the optical sensor system 140 of Fig. 2 comprising the light sources 142 as well as the optical sensor 144. The medical imaging system 100, e.g., comprises an exemplary magnetic resonance imaging system 152.

Alternatively, the medical imaging system 100 may, e.g., comprise a computed tomography system or an X-ray imaging system. The medical imaging system 100, e.g., with the MRI system 152, may be comprised by a radiation therapy (TR) system. An X-ray imaging system may, e.g., be part of an image guided therapy (IGT) system or the X-ray imaging system may, e.g., be a diagnostic X-ray (DXR) system.

A patient 118 is shown as being supported by a patient table 120. The patient table 120 is movable in at least two spatial directions 130, 132. For example, the patient table 120 is movable in a vertical direction 130 between a lower minimum position and an upper maximum position. For example, the patient table 120 is movable in a horizontal direction 132. By moving the patient table 120 in the vertical direction 132, the patient 118 lying on the patient table 120 is, e.g., moved into a magnet 154 of the MRI system 152. For example, the patient table 120 is movable in three spatial directions.

The MRI system 152 is controlled by the computer 400. The MRI system 152 comprises a magnet 154. The magnet 154 is a superconducting cylindrical type magnet with a bore 156 through it. The use of different types of magnets is also possible. For instance, it is also possible to use both a split cylindrical magnet and a so-called open magnet.

Within the bore 156 of the cylindrical magnet 154 there is an imaging iso-center 158 of MRI system 152. In the imaging iso-center 158, the magnetic field is, e.g., strong and uniform enough to perform magnetic resonance imaging.

Within the bore 156 of the magnet there is also a set of magnetic field gradient coils 160 which is used for acquisition of preliminary magnetic resonance data to spatially encode magnetic spins within the bore 156 of magnet 154. The magnetic field gradient coils 160 are connected to a magnetic field gradient coil power supply 162. The magnetic field gradient coils 160 are intended to be representative. Typically, magnetic field gradient coils 160 contain three separate sets of coils for spatially encoding in three orthogonal spatial directions. The magnetic field gradient power supply 162 supplies current to the magnetic field gradient coils 160. The current supplied to the magnetic field gradient coils 160 is controlled as a function of time and may be ramped or pulsed.

Furthermore, the MRI system 152 may comprise an RF transmitter coil 161 for manipulating the orientations of magnetic spins. The RF transmitter coil 161 may also be referred to as a transmitter antenna. The RF transmitter coil 161 is connected to an RF transmitter 163. It is understood that the RF transmitter coil 161 and the RF transmitter 163 are representative. The RF transmitter coil 161 may have multiple transmitter elements and the RF transmitter 163 may have multiple transmitter channels.

The transmitter 163, the gradient controller 162, the one or more lighting sources 142 the optical and the optical sensor 144 are shown as being connected to the hardware interface 406 of the computer 400. The computing device 400 is intended to represent one or more computational or computing devices. The computing device 400 is configured for acquiring medical imaging data as part of a control system of the medical imaging system 100, e.g., MRI images using the MRI system 152. The computing device 400 is further configured for controlling the one or more light sources 142 to adjust lighting conditions of a medical imaging system 100 during an acquisition of optical sensor data 414 using the optical sensor 144. The optical sensor 144 is configured for a supervision of the medical imaging system 100.

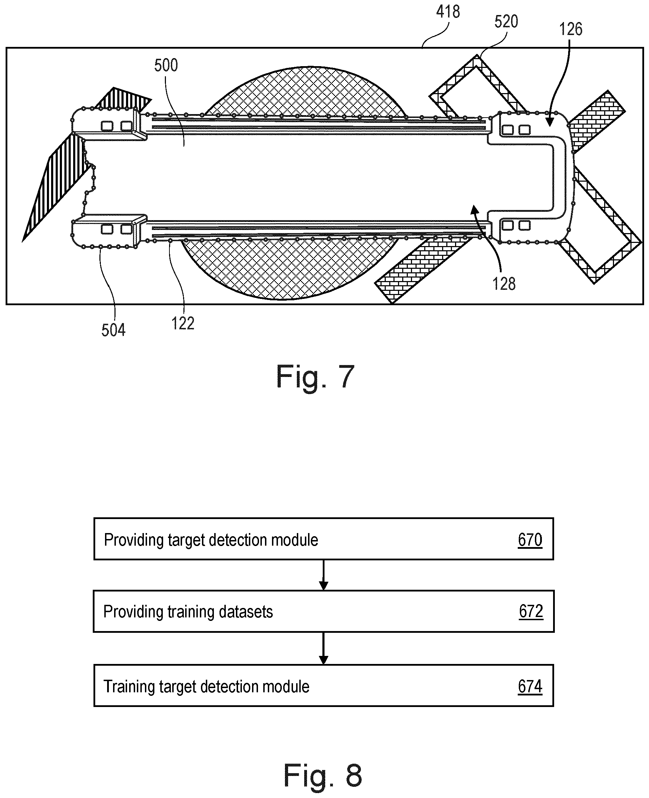

Fig. 7 illustrates exemplary optical sensor training data 418 for training a target detection module to detect target elements 122 of a patient table. Here, a keypoint element detection is used for detecting target elements 122 in for of keypoints or keypoint elements, i.e., keypoint features of the patient table, i.e., the target structure. Also, other detection methods, like bounding box detection or segmentation mask detection, may, e.g., be used. The optical sensor training data 418 is synthetic training data generated using a three-dimensional digital scale model 500 of the patient table. The three-dimensional digital scale model 500 comprises an upper surface 126 with a reclining surface 128. The target elements 122 in form of keypoint elements are, e.g., distributed on the upper surface 126. The target elements 122 in form of keypoint elements are, e.g., distributed along a contour line of the patient table circumferentially around the reclining surface 128. The optical sensor training data 418 may, e.g., be generated by adding random image elements 520. The random image elements 520 may, e.g., be added in the background of the three-dimensional digital scale model 500. For example, the random image elements 520 may also be added, at least partially, over the three-dimensional digital scale model 500 as random obstructions, such that at least some of the target elements 122 are covered by the random image elements 520. Thereby, the target detection module may be made robust against random obstructions, since a typical patient table may be covered additional material, like, e.g., with mattresses, paddings, coil interfaces and other material.

In addition, the optical sensor training data 418 comprises labels 504 labeling the target elements 122 within the optical sensor training data 418. For a training of the target detection module, e.g., additional optical sensor training data 418 may be provided without labels 504 of the target elements 122 as input for the target detection module during training. The target detection module is trained to detect in the optical sensor training data 418 may be provided without labels 504 the target elements 122, which are identified by the labels 504 of the labeled optical sensor training data 418.

For example, the labels 504 may comprise two-dimensional horizontal coordinate values of the respective target elements 122 of the patient table within the first coordinate system. The two-dimensional horizontal coordinate values may be descriptive of positions of the respective target elements in planes parallel to a sensor plane of the optical sensor system. Thus, the optical sensor training data 418 may, e.g., also be used for training the target detection module for determining the two-dimensional horizontal coordinate values of the labeled target elements 122 of the patient table as output.

For example, the labels 504 may further comprise third vertical coordinate values of the respective target elements 122 within the first coordinate system. The third vertical coordinate values are descriptive of distances of the positions of the respective target elements 122 from the sensor plane of the optical sensor system. Thus, the optical sensor training data 418 may, e.g., also be used for training the target detection module for determining the third vertical coordinate values in addition to the two-dimensional horizontal coordinate values of the labeled target elements 122 of the patient table as output.

Fig. 8 illustrates a method for training the target detection module to detect target elements within optical sensor data. In block 670, the target detection module to be trained is provided. In block 672, training datasets for training the target detection module are provided. The training datasets comprise optical sensor training data and optical sensor training data with target elements of the patient table labeled within the optical sensor training data. The target elements may, e.g., be labeled as keypoint elements. The target elements could also be identified and labeled within the optical sensor training data using bounding boxes or segmentation masks. The providing of the training datasets, e.g., comprises generating the optical sensor training data comprised by the training datasets as synthetic optical sensor training data using a three-dimensional digital scale model of the patient table. In block 674, the target detection module is trained for detecting as output the labeled target elements of the patient table within the optical sensor training data of the training datasets in response to receiving the optical sensor training data of the respective training dataset.

For example, the labels optical sensor training data may comprise two-dimensional horizontal coordinate values of the respective target elements of the patient table within the first coordinate system. The two-dimensional horizontal coordinate values may be descriptive of positions of the respective target elements in planes parallel to a sensor plane of the optical sensor system. The training may further comprise training the target detection module for determining as output the two-dimensional horizontal coordinate values of the labeled target elements of the patient table within the optical sensor training data of the training datasets in response to receiving the optical sensor training data of the respective training dataset.

For example, the labels may further comprise third vertical coordinate values of the respective target elements within the first coordinate system. The third vertical coordinate values are descriptive of distances of the positions of the respective target elements from the sensor plane of the optical sensor system. The training may further comprise training the target detection module for determining as output the third vertical coordinate values of the labeled target elements of the patient table within the optical sensor training data of the training datasets in response to receiving the optical sensor training data of the respective training dataset.

For example, the target detection module may be trained for determining confidence levels. These confidence levels may, e.g., be estimations trained using a loss function quantifying a deviation of the detections of the target elements from a ground truth for the target elements provided by the optical training data. The confidence levels may, e.g., be functions of an output of the target detection module. A maximum, minimum, mean or integral of the output of the detection module may be examples of quantities, which may be used to derive a confidence metric within a training distribution provided by a plurality of optical training data.

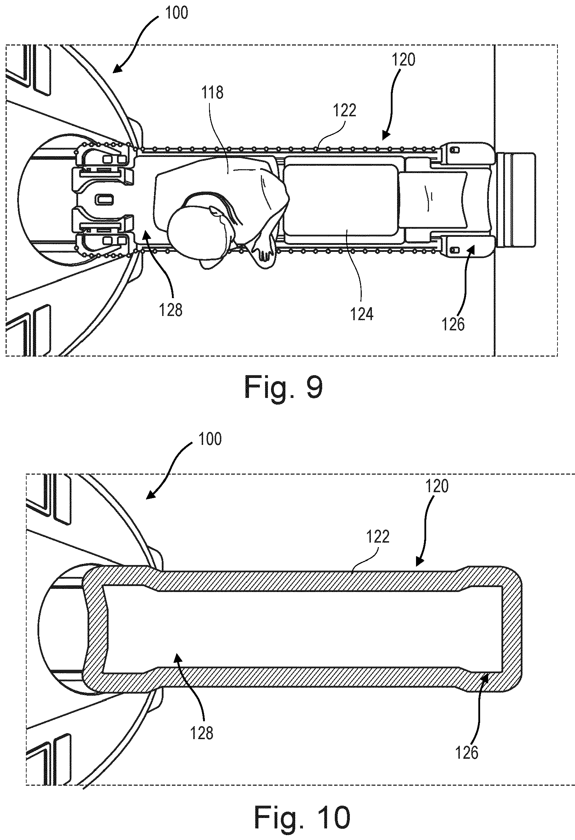

Fig. 9 illustrates an exemplary detection of target elements 122 using a keypoint detection optical sensor data 414 of a patient table 120. The patient table 120 is part of a medical imaging system, e.g., an MRI system. The patient table 120 comprises an upper surface 126 with a reclining surface 128. The reclining surface is configured for a patient to lie thereon. The target elements 122 to be detected by the target detection module are, e.g., target elements of the patient table 120. The target elements 122 of the patient table 120 in form of keypoint elements are, e.g., distributed on the upper surface 126. The target elements 122 of the patient table 120 in form of keypoint elements are, e.g., distributed along a contour line of the patient table circumferentially around the reclining surface 128. Furthermore, additional material 124, like, e.g., mattresses, paddings, blankets, coil interfaces and other material, may be arranged on the patient table 120. The target detection module is configured to detect the target elements 122 in the optical sensor data 414.

Fig. 10 illustrates another exemplary detection of target elements 122 using a keypoint detection in optical sensor data 414. The optical sensor data 414 of Fig. 9 comprises an empty patient table 120 of a medical imaging system 100 with an upper surface 126 with a reclining surface 128. The detected target elements 122 in form of keypoint elements are represented as gaussian probability heatmaps, e.g., with varying color overlaid over a table image. A size of the individual heatmaps may be such that neighboring target elements have overlapping signal tails. Due to the overlapping, the band-like structure schematically shown in Fig. 9 is formed, which represents the detected target elements 122.

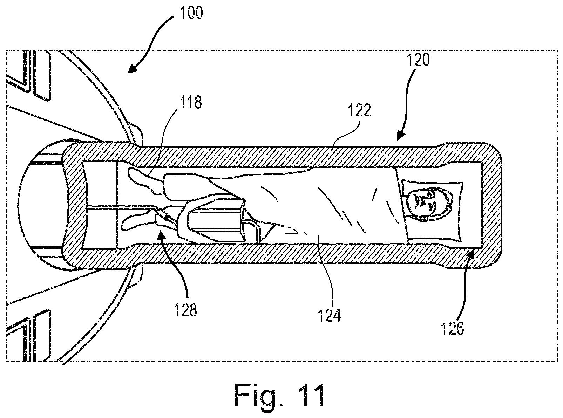

Fig. 11 illustrates another exemplary detection of target elements 122 using a keypoint detection in optical sensor data 414. The optical sensor data 414 of Fig. 10 corresponds to the optical sensor data 414 of Fig. 9, with the only difference, that patient table 120 in Fig. 10 is not empty. In Fig. 10, a patient is lying on the patient table 120. Furthermore, additional material 124, like, e.g., mattresses, paddings, blankets, coil interfaces and other material, are arranged on the patient table 120. This additional material 124 may, e.g., cover, at least partially, some of the target elements 122. Nevertheless, as indicated by the band-like structure schematically shown in Fig. 10, the target detection module is configured to robustly detect the target elements 122 in the optical sensor data 414.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical imaging system
- 118: patient
- 120: patient table
- 121: target area
- 122: target element
- 124: material arranged on patient table
- 125: anterior coil
- 126: upper surface
- 128: reclining surface
- 130: vertical direction
- 132: horizontal direction
- 140: optical sensor system
- 142: light source
- 144: optical sensor
- 152: MRI system
- 154: magnet
- 156: bore of magnet
- 158: imaging iso-center
- 160: magnetic field gradient coils
- 161: radio-frequency transmitter coil
- 162: magnetic field gradient coil power supply
- 163: transmitter
- 400: computing device
- 402: computational component
- 404: memory
- 406: hardware interface
- 408: user interface
- 410: machine readable instructions
- 412: target detection module
- 414: optical sensor data
- 416: control parameters
- 418: training data
- 500: 3D digital scale model
- 504: label
- 520: random image element

## Claims

1. A method for determining one or more control parameters (416) for controlling one or more light sources (142) to adjust lighting conditions of a medical imaging system (100) during an acquisition of optical sensor data (414) using an optical sensor (144), the optical sensor (144) being configured for a supervision of the medical imaging system (100), a computing device (400) used for executing the method comprising a target detection module (412) being configured for detecting target elements (122) of one or more target structures within optical sensor data (414) in response to receiving the optical sensor data (414) from the optical sensor (144),
the method comprising:
- receiving the optical sensor data (414),
- detecting the target elements (122) within the optical sensor data (414) using the target detection module (412),
- in response to receiving an adjustment trigger, starting a feedback loop, the feedback loop comprising:
controlling an adjusting of the lighting conditions of the medical imaging system (100) using the one or more light sources (142), receiving adjusted optical sensor data (414) for the adjusted lighting conditions from the optical sensor (144), detecting the target elements (122) within the adjusted optical sensor data (414) using the target detection module (412), and determining confidence values for the detections of the target elements (122) within the adjusted optical sensor data (414),
- the feedback loop being repeated until the confidence values determined for the detections of the target elements (122) within the adjusted optical sensor data (414) reach a predefined confidence threshold level, one or more adjustment parameters defining the adjusting of the lighting conditions, for which the confidence values reach a predefined confidence threshold level, being determined as the one or more control parameters (416).

2. The method of claim 1, the adjustment trigger comprising determining confidence values for the detections of the target elements (122) within the optical sensor data (414) below the predefined confidence threshold level.

3. The method of claim 2, the confidence values below the predefined confidence threshold level being determined for the detections of the target elements (122) within one or more spatial sections of interest within the optical sensor data (414).

4. The method of claim 1, the method further comprising determining quality parameters for the received optical sensor data (414), the adjustment trigger comprising detecting one or more of the following characteristics of the determined quality parameters: a signal-to-noise level below a predefined signal-to-noise level threshold, a total light intensity level below a predefined total light intensity threshold, a color-specific light intensity level below a predefined color-specific light intensity threshold, an intensity difference between different colors of light exceeding a predefined color intensity difference threshold.

5. The method of claim 4, the quality parameters being determined for one or more spatial sections of interest within the optical sensor data (414).

6. The method of any of the previous claims, the adjusting of the lighting conditions comprising one or more of the following: an increasing of a level of a total light intensity of an illumination by one or more of the light sources (142), an increasing of a color-specific light intensity of the illumination by one or more of the light sources (142), a shifting of a region of illumination by one or more of the light sources (142) spatially.

7. The method of any of the previous claims, the method further comprising applying one or more constraints to the adjusting of the lighting conditions, the one or more constraints comprising one or more of the following: an exclusion of one or more spatial sections within the optical sensor data (414) from the illumination by one or more of the light sources (142), a predefined maximum total light intensity threshold for the total light intensity of the illumination by one or more of the light sources (142), a predefined maximum color-specific light intensity threshold for a color-specific light intensity of the illumination by one or more of the light sources (142).

8. The method of any of the previous claims, an adjustment rate of the adjusting of the lighting conditions being lower than a predefined frame rate of optical images being provided using the optical sensor data (414) received from the optical sensor (144).

9. The method of any of claims 1 to 7, an adjustment rate of the adjusting of the lighting conditions being higher than the predefined frame rate of optical images.

10. The method of claim 9, the adjustment rate being higher than 30 Hz, preferably higher than 60 Hz.

11. The method of any of the previous claims, the method further comprising using the determined one or more control parameters (416) for controlling the light sources (142) for the supervision of the medical imaging system (100) using the optical sensor (144).

12. The method of claim 11, the light sources (142) being controlled to illuminate the medical imaging system (100) continuously using the one or more control parameters (416) independent of optical sensor data (414) acquisition by the optical sensor (144) or
the light sources (142) being controlled to synchronize the illumination of the medical imaging system (100) with the light sources (142) using the one or more control parameters (416) with the optical sensor data (414) acquisition by the optical sensor (144).

13. A computer program comprising machine executable instructions (410) for determining one or more control parameters (416) for controlling one or more light sources (142) to adjust lighting conditions of a medical imaging system (100) during an acquisition of optical sensor data (414) using an optical sensor (144), the optical sensor (144) being configured for a supervision of the medical imaging system (100), a target detection module (412) being configured for detecting target elements (122) of one or more target structures within optical sensor data (414) in response to receiving the optical sensor data (414) from the optical sensor (144),
execution of the machine executable instructions (410) by a processor (402) of a computing device (400) causing the processor (402) to control the computing device (400) to execute a method comprising:
- receiving the optical sensor data (414),
- detecting the target elements (122) within the optical sensor data (414) using the target detection module (412),
- in response to receiving an adjustment trigger, starting a feedback loop, the feedback loop comprising:
- controlling an adjusting of the lighting conditions of the medical imaging system (100) using the one or more light sources (142), receiving adjusted optical sensor data (414) for the adjusted lighting conditions from the optical sensor (144), detecting the target elements (122) within the adjusted optical sensor data (414) using the target detection module (412), and determining confidence values for the detections of the target elements (122) within the adjusted optical sensor data (414),
- the feedback loop being repeated until the confidence values determined for the detections of the target elements (122) within the adjusted optical sensor data (414) reach a predefined confidence threshold level, one or more adjustment parameters defining the adjusting of the lighting conditions, for which the confidence values reach a predefined confidence threshold level, being determined as the one or more control parameters (416).

14. A computing device (400) for determining one or more control parameters (416) for controlling one or more light sources (142) to adjust lighting conditions of a medical imaging system (100) during an acquisition of optical sensor data (414) using an optical sensor (144), the optical sensor (144) being configured for a supervision of the medical imaging system (100), the computing device (400) comprising a target detection module (412) being configured for detecting target elements (122) of one or more target structures within optical sensor data (414) in response to receiving the optical sensor data (414) from the optical sensor (144),
the computing device (400) comprising a processor (402) and a memory (404) with machine executable instructions (410) stored therein,
execution of the machine executable instructions (410) by the processor (402) of the computing device (400) causing the processor (402) to control the computing device (400) to execute a method comprising:
- receiving the optical sensor data (414),
- detecting the target elements (122) within the optical sensor data (414) using the target detection module (412),
- in response to receiving an adjustment trigger, starting a feedback loop, the feedback loop comprising:
controlling an adjusting of the lighting conditions of the medical imaging system (100) using the one or more light sources (142), receiving adjusted optical sensor data (414) for the adjusted lighting conditions from the optical sensor (144), detecting the target elements (122) within the adjusted optical sensor data (414) using the target detection module (412), and determining confidence values for the detections of the target elements (122) within the adjusted optical sensor data (414),
- the feedback loop being repeated until the confidence values determined for the detections of the target elements (122) within the adjusted optical sensor data (414) reach a predefined confidence threshold level, one or more adjustment parameters defining the adjusting of the lighting conditions, for which the confidence values reach a predefined confidence threshold level, being determined as the one or more control parameters (416).

15. A medical imaging system (100) comprising the computing device (400) of claim 14, one or more light sources (142) configured for illuminating the medical imaging system (100) and an optical sensor (144) configured for a supervision of the medical imaging system (100), the medical imaging system (100) being one of the following: a magnetic resonance imaging system (152), a computed tomography system, an X-ray imaging system.
